# EUROPÄISCHE PATENTANMELDUNG

(11) **EP 3 040 460 A1**
(43) Veröffentlichungstag der Anmeldung: **06.07.2016**
(21) Anmeldenummer: 15194095.4
(22) Anmeldetag: 11.11.2015
(51) Int. Cl.: D04B 21/02, A44B 18/00, A61F 13/62

(54) **LANDING ZONE MIT HOTMELTVERKLEBTEM VELOURS**

(30) Priorität: 30.12.2014 DE 102014119706
(71) Anmelder: Mondi Gronau GmbH, 48599 Gronau (DE)
(72) Erfinder: Homölle, Dieter, 48607 Ochtrup (DE); Baldauf, Georg, 48366 Laer (DE)
(74) Vertreter: Lorenz, Bernd Ingo Thaddeus

(57) **Zusammenfassung**

Die Erfindung betrifft ein Verfahren zur Herstellung eines Verbundstoffes für Klettverschlüsse, wobei ein Gewirke (1) bereitgestellt wird, wobei eine Trägerfolie (2) zugeführt wird, wobei ein Schmelzklebstoff (3) auf eine erste Seite des Gewirkes (1) aufgebracht wird und wobei das Gewirke (1) nachfolgend mit seiner ersten, mit Schmelzklebstoff (3) versehenen Seite auf eine erste Seite der Trägerfolie (2) aufgebracht und durch den Schmelzklebstoff (3) verklebt wird. Die Erfindung betrifft des Weiteren ein entsprechend gebildetes Verbundstoffelement.

## Beschreibung

Die Erfindung betrifft ein Verfahren zur Herstellung eines Verbundstoffelementes, welches ein Gewirke und eine Trägerfolie aufweist. Gegenstand der Erfindung ist des Weiteren ein solches Verbundstoffelement.

Der Verbundstoff bzw. ein davon abgetrenntes Verbundstoffelement sind als weiblicher Teil eines Klettverschlusses insbesondere für die Verwendung an Windeln oder Inkontinenzartikeln für Erwachsene vorgesehen.

Bei der Verwendung an Windeln wird das Verbundstoffelement auf dem vorderen Bündchenbereich der Windel angebracht. Mit Verschlussbändern, die seitlich an den Windeln befestigt sind und an ihrem freien Ende Kletthaken aufweisen, wird der Klettverschluss vervollständigt. Die einzelnen Kletthaken können dann in das Verbundstoffelement eingreifen und sich mit von dem Gewirke gebildeten Schlaufen verbinden.

Mit dem Klettverschluss kann erreicht werden, dass die Windel im Taillenbereich der die Windel tragenden Person gehalten ist. Klettverschlüsse können mehrfach geöffnet und verschlossen werden, ohne dass die Funktionalität des Verschlusses leidet. Im Gegensatz zu Klebeverschlüssen sind Klettverschlüsse unempfindlich gegenüber Kontakt mit Hautcremes oder Puder.

An ein Verbundstoffelement für einen Klettverschluss an einem Wegwerfprodukt wie z. B. Babywindeln oder vergleichbaren Inkontinenzartikeln für Erwachsene werden verschiedene Anforderungen gestellt. Zunächst muss das Verbundstoffelement ausreichende Verhakungsmöglichkeiten für die Kletthaken bilden und auch eine ausreichende Haltekraft gewährleisten.

Sowohl das Gewirke als auch die Trägerfolie sollen des Weiteren trotz der ausreichenden Haltekräfte ein möglichst geringes Flächengewicht aufweisen, damit eine kostengünstige Produktion möglich ist. Üblicherweise ist auch im Bereich des Verbundstoffelementes ein Aufdruck vorgesehen, weshalb zumindest das außenliegende Gewirke durchscheinend sein soll, damit ein darunterliegender Aufdruck von einem Benutzer sichtbar ist.

Schließlich wird auch eine möglichst weiche textilartige Oberfläche angestrebt, weil diese für einen Benutzer angenehmer ist und besonders hochwertig wirkt.

Gerade bei den aus Kostengründen angestrebten niedrigen Flächengewichten ist die Verklebung des Gewirkes mit der Trägerfolie für die Funktionalität des Verbundstoffelementes ausschlaggebend. In der Praxis wird üblicherweise ein einkomponentiger PUR-Klebstoff auf die Trägerfolie aufgebracht, wobei dann auf die Trägerfolie mit dem aufgebrachten Klebstoff das Gewirke aufgeklebt wird. Dabei ist zu beachten, dass der Klebstoff auch die einzelnen Schlaufen verkleben kann, wodurch dann die Funktionalität des Verbundstoffelementes als Teil eines Klettverschlusses beeinträchtigt ist.

Vor diesem Hintergrund sind aus dem Stand der Technik verschiedene Ansätze bekannt, um bei der beschriebenen Verfahrensweise eine gute Klettwirkung zu erreichen.

So ist aus EP 0 777 006 B1 ein Verbundstoff für Klettverschlüsse bekannt, bei dem das textile Substrat aus einem Gewirke aus Kett- und Schussfäden sowie wirktechnisch mit dem Gewirke verbundenen Schlaufen gebildet ist. Das textile Substrat ist mit der Trägerfolie verklebt. Die Schlaufen sind so groß bemessen, dass sie auf den von dem Grundgewebe gebildeten Maschen aufliegen. Durch die Bemessung der Schlaufen soll erreicht werden, dass die Schlaufen nicht mit dem Klebstoff in Verbindung kommen und ihre Funktionalität behalten. Das zuvor erläuterte Problem, bei der Verwendung eines offenen textilen Substrates sowohl eine gute Klettwirkung als auch eine hohe Verbundfestigkeit zwischen Trägerfolie und textilem Substrat sicherzustellen, ist jedoch noch nicht in vollem Umfang gelöst. Insbesondere die Verbindung zwischen Trägerfolie und textilem Substrat ist noch verbesserungsbedürftig.

Gemäß EP 1 579 779 B1, EP 2 439 323 A1 und WO 2006/045118 A1 ist es bekannt, den Klebstoff in einem Muster auf die Trägerfolie aufzubringen, so dass dann das Gewirke lediglich bereichsweise verklebt ist, wobei klebstofffreie Bereiche verbleiben. In den klebstofffreien Bereichen kann eine Beeinträchtigung der Klettwirkung durch den Klebstoff ausgeschlossen werden. Gleichzeitig kann der Klebstoff in dem Klebstoffmuster so aufgetragen werden, dass lokal eine sichere Verklebung erreicht wird. Dort wo der Klebstoff aufgetragen ist, kann auch in einem gewissen Maße eine Verklebung der für die Verbindung mit den Kletthaken vorgesehen Schlaufen hingenommen werden.

Um zusätzlich ein Abreißen des Gewirkes von der Trägerfolie zu vermeiden, ist gemäß der EP 2 439 323 A1 an dem Rand der einzelnen Verbundstoffelemente ein umlaufender Klebstoffrahmen vorgesehen. Die EP 2 439 323 A1 zeigt des Weiteren auch schematisch die bevorzugte Struktur des Gewirkes, welches als Fransen gewirkte Maschenstäbchen, Verbindungsfäden zwischen den Maschenstäbchen sowie schlaufenbildende Fäden umfasst. Die Schlaufen können dadurch bereitgestellt werden, dass die entsprechenden Fäden bei dem Wirkprozess mit einer niedrigen Zugspannung bzw. ohne Zugspannung verarbeitet werden. Das Gewirke wird auch als "Lock Loop"-Gewirke bezeichnet.

Durch die beschriebenen Verbesserungen und insbesondere die Optimierung des Klebstoffmusters können kostengünstige Verbundstoffelemente bereitgestellt werden, welche ausreichende Kletteigenschaften aufweisen. Entsprechend haben sich derartige Produkte auch im Markt bewährt. Das Flächengewicht des Gewirkes kann beispielsweise zwischen 18 und 25 g/m² liegen, wobei das beschriebene "Lock Loop"-Gewirke mit einer 14 µm dicken Folie aus Polyethylen durch einen einkomponentigen PUR-Klebstoff verbunden ist. Der einkomponentige PUR-Klebstoff härtet durch Feuchtigkeit aus, wozu entweder die Luftfeuchtigkeit ausreichend ist oder auch zusätzlich eine Berieselung mit Wasser erfolgen kann. Die Trägerfolie aus Polyethylen ist üblicherweise an ihrer dem Gewirke gegenüberliegenden Oberseite mit einem Aufdruck versehen.

Ausgehend von einem solchen bekannten Verbundstoff bzw. Verbundstoffelement liegt der vorliegenden Erfindung die Aufgabe zugrunde, ohne einen übermäßen Anstieg der Produktionskosten verbesserte mechanische Eigenschaften und insbesondere eine weiche Oberfläche zu erreichen.

Gegenstand der Erfindung und Lösung der Aufgabe sind ein Verfahren zur Herstellung eines Verbundstoffes für Klettverschlüsse gemäß Patentanspruch 1 sowie ein Verbundstoffelement für Klettverschlüsse gemäß Patentanspruch 11.

Im Rahmen der Erfindung ist ein Verfahren zur Herstellung eines Verbundstoffelementes für Klettverschlüsse vorgesehen, bei dem ein Gewirke bereitgestellt und eine Trägerfolie zugeführt wird, wobei ein Schmelzklebstoff auf eine erste Seite des Gewirkes aufgebracht wird und wobei das Gewirke nachfolgend mit seiner ersten, mit Schmelzklebstoff versehenen Seite auf eine erste Seite der Trägerfolie aufgebracht und durch den Schmelzklebstoff verklebt wird.

Erfindungsgemäß wird ein Schmelzklebstoff verwendet, der nicht auf die Trägerfolie sondern auf das Gewirke aufgebracht wird. Der Schmelzklebstoff kann durch eine geeignete Temperatursteuerung in seiner Viskosität genau eingestellt werden und wird direkt auf das Gewirke aufgebracht. Im Gegensatz zu einem Auftrag des Klebstoffes auf die Trägerfolie ist der Schmelzklebstoff nur genau dort vorhanden, wo sich die Fäden des Gewirkes befinden, so dass sich eine effizientere Verklebung ergibt, während bei einem Auftrag des Klebstoffes auf die Trägerfolie stets auch von dem Gewirke freigelassene Abschnitte und Zwischenräume mit Klebstoff versehen sind. Als weiterer Vorteil ergibt sich, dass genau die Seite des Gewirkes, welche mit der Trägerfolie verklebt werden soll, mit Schmelzklebstoff versehen wird, während die an der gegenüberliegenden Seite freiliegenden Schlaufen nicht mit eingebunden werden. Der Schmelzklebstoff kann in seiner Viskosität insbesondere so eingestellt werden, dass dieser sich nicht unkontrolliert in dem Gewirke verteilt, wozu der Schmelzklebstoff vorzugsweise leicht zähflüssig verarbeitet wird.

Der Schmelzklebstoff wird vorzugsweise mit einem Flächengewicht zwischen 2 g/m² und 5 g/m² auf die erste Seite des Gewirkes aufgebracht. Wie bereits zuvor beschrieben, ergibt sich der Vorteil, dass der Schmelzklebstoff dann an einzelnen Fäden des Gewirkes anhaftet und dort dann im ausreichenden Maße vorhanden ist. An der gegenüberliegenden Seite vorstehende Fäden oder auch Zwischenräume können nicht mit Schmelzklebstoff versehen werden, so dass sich eine besonders effiziente Ausnutzung des Klebstoffes ergibt.

Der Schmelzklebstoff kann beispielsweise mit einer Walze aufgetragen werden, die vollflächig mit Klebstoff versehen ist, so das dann das Gewirke auf der gesamten Fläche der Walze den Klebstoff abnehmen kann. Darüber hinaus kann der Schmelzklebstoff auf der Walze aber auch in einem Muster angeordnet sein, um wie aus der EP 2 439 323 A1 bekannt eine weitere Optimierung zu erzielen. Da aber im Rahmen der vorliegenden Erfindung im Gegensatz zu dem Stand der Technik der Schmelzklebstoff zunächst von der entsprechenden Seite des Gewirkes aufgenommen wird und damit Zwischenräume sowie die gegenüberliegende Seite des Gewirkes frei von Schmelzklebstoff bleiben, kann auch ohne Weiteres der Schmelzklebstoff vollflächig bereitgestellt werden.

Des Weiteren ist es auch möglich, den Schmelzklebstoff von oben in einem Muster oder vollflächig mit einer Extrusionsdüse auf das Gewirke aufzubringen. Auch dann ergibt sich der Vorteil, dass bei einer entsprechenden Zähflüssigkeit des Schmelzklebstoffes der Schmelzklebstoff nicht zu tief in das Gewirke gelangt und nicht auf der gegenüberliegenden Seite die dort gebildeten freien Schlaufen einbinden kann.

Wie zuvor beschrieben, ist auch die Viskosität des Schmelzklebstoffes bei seiner Verarbeitung von Bedeutung. So liegt die dynamische Viskosität η bei der Verarbeitungstemperatur vorzugsweise zwischen 2 Pa · s und 6 Pa · s. In dem beschriebenen Bereich wird erreicht, dass der Schmelzklebstoff gut verarbeitet werden kann, an dem Gewirke anhaftet und eine Verklebung mit der Trägerfolie ermöglicht, ohne dass der Schmelzklebstoff sich in dem Gewirke zu stark verteilt.

Im Rahmen der Erfindung kann beispielsweise ein Schmelzklebstoff auf der Basis von Styrol-Isopren-Styrol-Copolymer (SIS) eingesetzt werden. Die Verarbeitungstemperatur kann beispielsweise zwischen 150°C bis 170°C liegen. Besonders bevorzugt weist der Schmelzklebstoff bei seiner Verarbeitung eine Viskosität zwischen 3 Pa · s und 5 Pa · s auf, wobei dann die Verarbeitungstemperatur insbesondere zwischen 155°C und 165°C liegen kann.

Als weitere Maßnahme kann vorgesehen sein, dass bei einem Auftrag des Schmelzklebstoffes über eine Walze das Gewirke an der Oberseite der Walze geführt wird, so dass dann der Schmelzklebstoff sich an der Unterseite des Gewirkes befindet, wodurch dann auch durch die Gewichtskraft der Schmelzklebstoff nicht tiefer in das Gewirke eindringen kann und eine unerwünschte Verklebung freier Schlaufen verhindert wird.

Gemäß einer bevorzugten Ausgestaltung der Erfindung wird als Gewirke ein schlaufenbildendes Veloursgewirke bereitgestellt. Dabei handelt es sich um ein mit zwei Legeschienen hergestelltes Gewirke, welches freie vorstehende Schlaufen aufweist.

Vorzugsweise wird das Gewirke nach der Maschenbildung, also dem eigentlichen Wirkprozess im engen Sinne, und vor einer thermischen Fixierung zur Bildung und Ausrichtung der Schlaufen gebürstet, bevor nachfolgend der Schmelzklebstoff aufgebracht wird. Die thermische Fixierung wird in der Praxis auch als Ausrüstung bezeichnet und ist bei textilen Materialien aus Kunststoff üblich. Bei der thermischen Fixierung wird das Material so weit erwärmt, dass die einzelnen Fäden zwar nicht aufschmelzen, innere Spannungen durch ein zähes Fließen der Polymerketten jedoch reduziert werden.

Durch das Bürsten werden also zunächst die Schlaufen gebildet, wobei das Material sich auch in einem gewissen Maße zusammenziehen kann. Die so erhaltene Struktur wird dann durch die Ausrüstung dauerhaft fixiert.

Besonders bevorzugt wird das Veloursgewirke mit einer ersten Legeschiene und einer zweiten Legeschiene gewirkt, wobei die erste Legeschiene in einem Muster 1-0/1-2 geführt wird und wobei die zweite Legeschiene in einem Muster ausgewählt aus der Gruppe 1-0/2-3, 1-0/3-4 und 1-0/4-5 geführt wird. Ein solches auch als "Loop-Raised Fabric" bezeichnetes Veloursgewebe ist beispielsweise aus dem Fachbuch "Warp Knitting Technology, D. F. Paling, 2. Auflage 1965", Seiten 100 und 101 bekannt.

Das Veloursgewirke zeichnet sich durch einen hohe Weichheit, ein hohes Volumen sowie auch verbesserte mechanische Eigenschaften aus. Es wird angenommen, dass dieser Vorteil zumindest teilweise auch darauf zurückzuführen ist, dass die einzelnen von dem Veloursgewirke gebildeten Schlaufen sich aufgrund des Legemusters über mehrere Maschenstäbchen erstrecken.

Vorzugsweise liegt das Flächengewicht des Gewirkes zwischen 25 g/m² und 40 g/m².

Gemäß einer bevorzugten Ausgestaltung der Erfindung ist vorgesehen, dass die Trägerfolie vor dem Verkleben mit dem Gewirke an der ersten Seite bedruckt wird. Der Aufdruck auf die Trägerfolie erfolgt also dort, wo nachfolgend auch das Gewirke aufgeklebt wird. Es ergibt sich der Vorteil, dass dann der Aufdruck lediglich durch das Gewirke hindurch und nicht durch das Gewirke und die Trägerfolie sichtbar ist, so dass der Aufdruck besser erkennbar ist und eine höhere Qualität aufweist.

Als Material für das Gewirke sind insbesondere Polyamid und Polyester geeignet. Gemäß einer besonders bevorzugten Ausgestaltung der Erfindung ist das Gewirke aus einer ersten Gruppe von Fäden aus Polyamid und einer zweiten Gruppe aus Fäden aus Polyester, insbesondere Polyethylenterephthalat (PET) gebildet. Jede der beiden Gruppen ist dann jeweils eine der beiden Legeschienen zugeordnet.

Die Feinheit der Fäden liegt vorzugsweise zwischen 15 und 45 dtex, besonders bevorzugt zwischen 20 und 35 dtex, wobei auch ein Multifilamentgarn verwendet werden kann.

Gegenstand der Erfindung ist auch ein Verbundstoffelement für Klettverschlüsse mit einer Trägerfolie und einem mit der Trägerfolie verklebten, schlaufenbildenden Gewirke mit einem Flächengewicht zwischen 20 und 60 g/m². Erfindungsgemäß ist das Gewirke ein Veloursgewirke, wobei das Gewirke und die Trägerfolie durch einen auf das Gewirke aufgebrachten Schmelzklebstoff verklebt sind. Die Aufbringung des Schmelzklebstoffes auf das Gewirke ist auch bei dem fertigen Verbundstoffelement unmittelbar erkennbar, weil an Zwischenräumen des Gewirkes kein oder Klebstoff vorhanden ist und der Klebstoff an den Fäden des Gewirkes anhaftet.

Wie bereits zuvor beschrieben weist das Verbundstoffelement auf der Trägerfolie an der mit dem Gewirke verklebten Seite bevorzugt einen Aufdruck auf, der dann durch das Gewirke gut sichtbar ist.

Die Trägerfolie kann eine Dicke zwischen 8 µm und 50 µm, vorzugsweise zwischen 10 µm und 20 µm aufweisen. Geeignet sind insbesondere Trägerfolien aus Polyolefin, beispielsweise Polyethylen (PE).

Die Erfindung wird im Folgenden anhand einer lediglich ein Ausführungsbeispiel darstellenden Zeichnung erläutert. Es zeigen:
- **Fig. 1**: Den Schichtaufbau eines Verbundstoffelementes für Klettverschlüsse in einer rein schematischen Ansicht,
- **Fig. 2**: eine schematische Detailansicht einer Verklebung eines Gewirkes des Verbundstoffelementes mit einer Trägerfolie,
- **Fig. 3**: Verfahrensschritte zur Bereitstellung des Gewirkes
- Fig. 4a und 4b: alternative Varianten zur Herstellung eines Verbundstoffes,
- **Fig. 5**: das Maschinenbild eines Veloursgewirkes vor einem Bürsten und Thermofixieren.

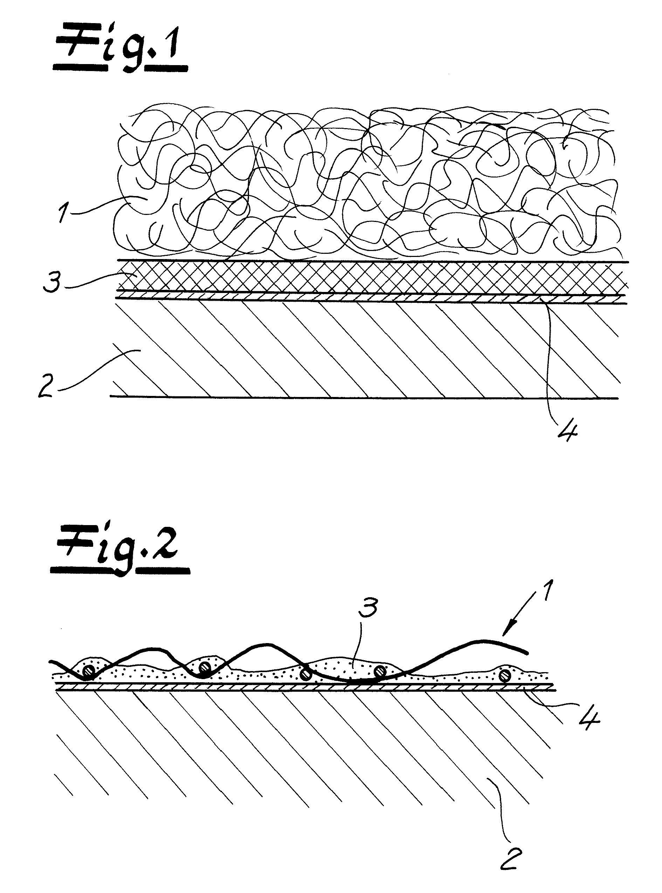

Die Fig. 1 zeigt rein schematisch den Schichtaufbau eines Verbundstoffelementes mit einem Gewirke 1 und einer Trägerfolie 2, die durch einen Schmelzklebstoff 3 auf der Basis von SIS verklebt sind. Des Weiteren ist zu erkennen, dass die Trägerfolie 2 an der mit dem Gewirke 1 verbundenen Seite einen Aufdruck 4 aufweist.

Das dargestellte Verbundstoffelement bildet den weiblichen Teil eines Klettverschlusses, wobei die Trägerfolie 2 mit der dem Gewirke 1 gegenüberliegenden Seite auf den vorderen Taillenbereich einer Windel aufgebracht werden kann. An der von dem Gewirke 1 gebildeten Außenseite ist dann der Aufdruck 4 durch das Gewirke 1 hindurch sichtbar. In dem dargestellten Ausführungsbeispiel ist die Trägerfolie 2 aus Polyethylen gebildet und weist eine Dicke von 14 µm auf.

Wie nachfolgend noch im Detail erläutert, handelt es sich bei dem Gewirke 1 um ein Veloursgewirke mit einem Flächengewicht von 25 g/m². Der Schmelzklebstoff ist mit einem Flächengewicht zwischen 3 bis 5 g/m² auf dem Aufdruck 4 vorgesehen.

Erfindungsgemäß wird der Schmelzklebstoff 3 bei der nachfolgend noch im Detail beschriebenen Herstellung auf das Gewirke 1 aufgebracht. Entsprechend haftet der Schmelzklebstoff 3 zunächst an den einzelnen Fäden des Gewirkes 1, so dass letztlich eine Verbindung der einzelnen Fäden mit der Trägerfolie 2 gemäß der Fig. 2 erfolgt. Zwischenräume des Gewirkes 1 sowie aus der Ebene vorstehende Fäden des Gewirkes 1 werden nicht verklebt.

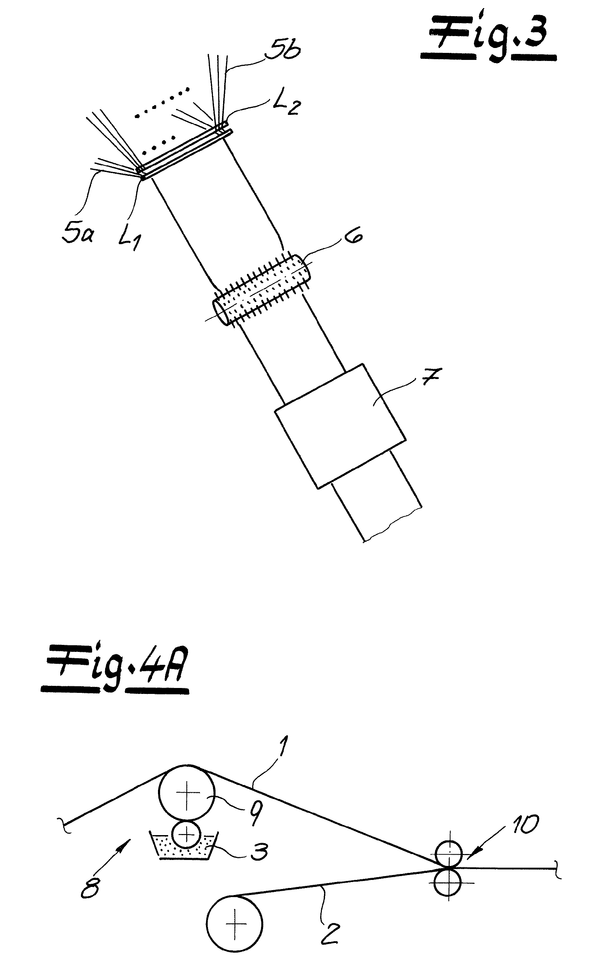

In Fig. 3 sind Verfahrensschritte zur Bereitstellung des Gewirkes dargestellt.

Zunächst wird das Gewirke 1 mit einer ersten Legeschiene L₁ und einer zweiten Legeschiene L₂ als Veloursgewirke gebildet. Die erste Legeschiene L₁ wird dabei in einem Muster 1-0/1-2 geführt, während die zweite Legeschiene in einem Muster ausgewählt aus der Gruppe 1-0/2-3, 1-0/3-4 und 1-0/4-5 geführt wird.

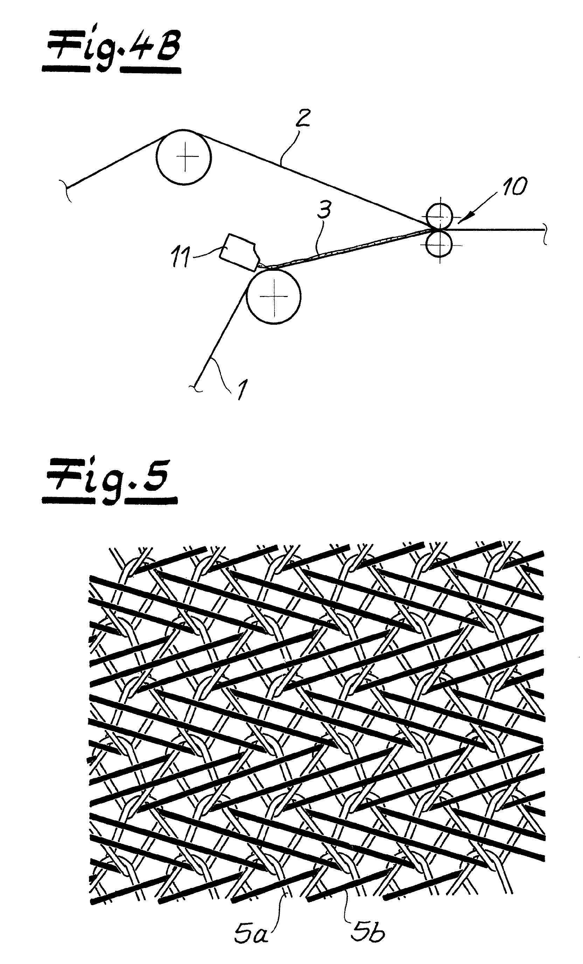

Es entsteht dadurch das in der Fig. 5 dargestellte Veloursgewebe mit einer ersten Gruppe von Fäden 5a und zweiten Gruppe von Fäden 5b.

Nachfolgend wird das so gebildete Veloursgewirke gebürstet, wozu in Fig. 3 exemplarisch eine Bürstenwalze 6 dargestellt ist. Bei dem Bürsten werden dann aus den Fäden 5b der zweiten Gruppe Schlaufen gebildet und aufgerichtet.

Sodann wird das Veloursgewirke in einer Fixiereinrichtung 7 thermofixiert.

Das so vorbereitete Gewirke 1 kann dann gemäß der Fig. 4a an seiner Unterseite mit dem Schmelzklebstoff 3 versehen. Der Schmelzklebstoff 3 kann beispielsweise über eine Auftragvorrichtung 8 aufgebracht werden, welche eine Transferwalze 9 umfasst. Das Gewirke 1 nimmt dann den Schmelzklebstoff 3 von der Transferwalze 9 ab, so dass nur die untenliegenden Fäden des Gewirkes 1 mit Schmelzklebstoff 3 versehen sind. Der Schmelzklebstoff 3 wird mit einem mittleren Flächengewicht zwischen 2 bis 5 g/m² aufgebracht, wobei insbesondere bei einem Schmelzklebstoff 3 auf der Basis SIS die Verarbeitungstemperatur zwischen 150°C bis 170°C, insbesondere zwischen 155°C bis 165°C liegen kann. Die Viskosität liegt vorzugsweise zwischen 2 Pa · s und 6 Pa · s, besonders bevorzugt zwischen 3 Pa · s und 5 Pa · s. Dadurch wird erreicht, dass der Schmelzklebstoff 3 gut an dem Gewirke 1 anhaftet und dann auch eine Verbindung mit der Trägerfolie 2 ermöglicht, sich jedoch nicht übermäßig in dem Gewirke 1 verteilt.

Die Fig. 4b zeigt eine alternative Variante zur Bildung des Verbundstoffes, wobei die Trägerfolie 2 zu dem Walzenspalt 10 von oben und das Gewirke 1 von unten zugeführt werden. Der Schmelzklebstoff 3 wird dann von oben auf das Gewirke 1 mit einer Extrusionsdüse 11 vollflächig aufgebracht, wobei auf dem Gewirke 1 eine Schicht entsteht, die sich innig mit den Fäden des Gewirkes verbindet und sich an den einzelnen Fäden auch ansammelt, wobei die Viskosität des Schmelzklebstoffes 3 jedoch so eingestellt ist, dass der Schmelzklebstoff 3 nicht zu tief in das Gewirke eindringt, so dass die bei der Darstellung gemäß der Fig. 4b untenliegenden Schlaufen nicht mit eingebunden werden. Zwischen der Extrusionsdüse 11 und dem Gewirke 1 wird zweckmäßigerweise ein geringer Abstand vorgesehen, damit eine direkte Berührung zwischen der Extrusionsdüse 11 und dem Gewirke 1 vermieden wird. Der Abstand von der Extrusionsdüse 11 zu dem Walzenspalt 10 ist so einzustellen, dass der Schmelzklebstoff 3 sich nicht zu stark abkühlt.

Gemäß der Fig. 3 werden dann das mit dem Schmelzklebstoff 3 versehene Gewirke 1 und die Trägerfolie 2 in einem Walzenspalt 10 miteinander verbunden. Nicht dargestellt ist, dass die Trägerfolie 2 vor der Verbindung mit dem Gewirke 1 in herkömmlicher Weise mit einem Aufdruck 4 versehen werden kann.

Im Rahmen orientierender Versuche wurde ein erfindungsgemäßes Verbundstoffelement mit Vergleichsbeispielen verglichen, wobei mit zwei unterschiedlichen Hakenmaterialien 3M Global Hook und Aplix 980 die Haltekräfte (Peel) bestimmt wurden. Das Verbundstoffelement wurde dazu auf einen Klebestreifen aufgebracht, um eine ausreichende mechanische Festigkeit zu erreichen.

Die bestimmte Peel-Festigkeit kennzeichnet das Haften und Abschälen des Hakenmaterials auf dem Verbundstoffelement, nachdem das Hakenmaterial mit einem spezifischen Gewicht auf das Verbundstoffelement aufgedrückt und anschließend abgeschält wird. Die Prüfung erfolgt in einem Zugversuch mit einem Zwick-Gerät, wobei die beiden Enden der Probe (Hakenmaterial einerseits sowie Verbundstoffelement andererseits) in die Spannbacken des Zwickgerätes eingespannt werden. Sowohl das Hakenmaterial als auch das Verbundstoffelement werden mit einer Probenbreite von 25,4 mm bereitgestellt. Die Prüfung erfolgt bei einer Umgebungstemperatur von 23°C ± 2°C und einer Luftfeuchtigkeit von 50 % ± 2 %, wobei diese Parameter über zumindest 2 Stunden konstant zu halten sind. Um gleichmäßige Prüfbedingungen zu erreichen, werden das Hakenband und das Verbundstoffelement zwischen Daumen und Zeigefinger für 3 Sekunden. zusammengedrückt, bevor dann die Probe für 5 Sekunden mit einem Hängegewicht von 500 g beaufschlagt wird. Nach der Entfernung des Gewichtes wird dann die für ein Abziehen notwendige Kraft pro Inch (Peel-Festigkeit) bestimmt.

Die Ergebnisse der Untersuchung sind in der Tabelle 1

| **Tabelle 1** | Peel-Wert 3M Global Hook in N/Inch | Peel-Wert Aplix 980 in N/Inch | Soft touch |
|---|---|---|---|
| Erfindungsgemäß | 3,9 | 3,4 | sehr gut |
| Vergleichsbeispiel 1 | 2,3 | 2,2 | ausreichend |
| Vergleichsbeispiel 2 | 2,0 | 1,6 | befriedigend |
| Vergleichsbeispiel 3 | 2,6 | 2,5 | befriedigend |

dargestellt, wobei sich unabhängig von der genauen Durchführung der Versuche klare qualitative Unterschiede ableiten lassen. Bei dem erfindungsgemäßen Verbundstoffelement wurde ein Gewirke 1 in Form eines Veloursgewirkes mit einem Flächengewicht von 35 g/m² mit Schmelzklebstoff bei einem Flächengewicht des Schmelzklebstoffes von 3,5 g/m² beschichtet und mit der Trägerfolie 2 kaschiert. In dem Vergleichsbeispiel 1 wird ein aus dem Markt bekanntes Standard-Verbundstoffelement eingesetzt, wobei ein Gewirke gemäß der EP 2 439 323 A1 mit einem Flächengewicht von 18 g/m² mit einem Einkomponenten-PUR-Klebstoff auf die Trägerfolie 2 kaschiert wird. Der Auftrag des Klebstoffes erfolgt in einem Klebstoffmuster.

Bei dem Vergleichsbeispiel 2 wird eine Veloursgewirke gemäß der vorliegenden Erfindung mit dem einkomponentigen PUR-Klebstoff in einem Klebstoffmuster beim Auftragsgewicht von 2 g/m² mit der Trägerfolie 2 kaschiert. Gemäß dem Vergleichsbeispiel 3 erfolgt dagegen bei einem gleich ausgebildeten Gewirke eine vollflächige Verklebung mit dem einkomponentigen PUR-Klebstoff und dem Auftragsgewicht von 2 g/m².

Gemäß der Tabelle 1 ist ersichtlich, dass sich das erfindungsgemäße Verbundstoffelement durch sehr gute mechanische Eigenschaften auszeichnet. Darüber hinaus ergibt sich auch eine besonders weiche und für einen Benutzer angenehme Haptik, die in der Tabelle als "Soft touch" bezeichnet ist.

## Patentansprüche

1. Verfahren zur Herstellung eines Verbundstoffes für Klettverschlüsse,
wobei ein Gewirke (1) bereitgestellt wird,
wobei eine Trägerfolie (2) zugeführt wird,
wobei ein Schmelzklebstoff (3) auf eine erste Seite des Gewirkes (1) aufgebracht wird,
wobei das Gewirke (1) nachfolgend mit seiner ersten, mit Schmelzklebstoff (3) versehenen Seite auf eine erste Seite der Trägerfolie (2) aufgebracht und durch den Schmelzklebstoff (3) verklebt wird.

2. Verfahren nach Anspruch 1, wobei der Schmelzklebstoff (3) mit einem Flächengewicht zwischen 2 bis 5 g/m² auf die erste Seite des Gewirkes (1) aufgebracht wird.

3. Verfahren nach Anspruch 1 oder 2, wobei der Schmelzklebstoff (3) mit einer Transferwalze (9) aufgetragen wird.

4. Verfahren nach einem der Ansprüche 1 bis 3, wobei der Schmelzklebstoff (3) mit einer Viskosität zwischen 2 Pa · s und 6 Pa · s auf die erste Seite des Gewirkes (1) aufgebracht wird.

5. Verfahren nach einem der Ansprüche 1 bis 4, wobei als Gewirke (1) ein schlaufenbildendes Veloursgewirke bereitgestellt wird.

6. Verfahren nach Anspruch 5, wobei das Veloursgewirke mit einer ersten Legeschiene (L₁) und einer zweiten Legeschiene (L₂) gewirkt wird, wobei die erste Legeschiene (L₁) in einem Muster 1-0/1-2 geführt wird und wobei die zweite Legeschiene (L₂) in einem Muster ausgewählt aus der Gruppe 1-0/2-3, 1-0/3-4 und 1-0/4-5 geführt wird.

7. Verfahren nach Anspruch 5 oder 6, wobei das Gewirke (1) nach der Maschenbildung und vor einer thermischen Fixierung zur Bildung und Aufrichtung der Schlaufen gebürstet wird, bevor nachfolgend der Schmelzklebstoff (3) aufgebracht wird.

8. Verfahren nach einem der Ansprüche 1 bis 7, wobei das Gewirke (1) mit einem Flächengewicht zwischen 25 g/m² und 60 g/m² bereitgestellt wird.

9. Verfahren nach einem der Ansprüche 1 bis 8, **dadurch gekennzeichnet, dass** die Trägerfolie (2) vor dem Verkleben mit dem Gewirke (1) an der ersten Seite bedruckt wird.

10. Verfahren nach einem der Ansprüche 1 bis 9, wobei das Gewirke (1) aus einer ersten Gruppe von Fäden aus Polyamid und einer zweiten Gruppe von Fäden aus Polyester gebildet ist.

11. Verbundstoffelement für Klettverschlüsse mit einer Trägerfolie (2) und einem mit der Trägerfolie (2) verklebten, schlaufenbildenden Gewirke (1) mit einem Flächengewicht zwischen 20 und 60 g/m², **dadurch gekennzeichnet, dass** das Gewirke (1) ein Veloursgewirke ist und dass das Gewirke (1) und die Trägerfolie (2) durch einen auf das Gewirke (1) aufgebrachten Schmelzklebstoff (3) verklebt sind.

12. Verbundstoffelement nach Anspruch 11, **dadurch gekennzeichnet, dass** die Trägerfolie (2) an ihrer mit dem Gewirke (1) verklebten Seite einen Aufdruck (4) aufweist.
